(11) **EP 2 336 751 B1**

(12) **EUROPÄISCHE PATENTSCHRIFT**

(45) Veröffentlichungstag und Bekanntmachung des Hinweises auf die Patenterteilung:
**10.09.2014   Patentblatt 2014/37**

(51) Int Cl.:
**G01N 21/64** *(2006.01)*     **G01N 33/08** *(2006.01)*
**A01K 45/00** *(2006.01)*

(21) Anmeldenummer: **09015586.2**

(22) Anmeldetag: **16.12.2009**

(54) **Verfahren zur Bestimmung des Geschlechts an Vogeleiern**

Method for determining the gender of bird eggs

Procédé de détermination du sexe d'oeufs d'oiseaux

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(43) Veröffentlichungstag der Anmeldung:
**22.06.2011   Patentblatt 2011/25**

(73) Patentinhaber: **Fraunhofer-Gesellschaft zur Förderung der angewandten Forschung e.V.
80686 München (DE)**

(72) Erfinder:
• **Opitz, Jörg, Dr.
01219 Dresden (DE)**

• **Fischer, Björn
09212 Limbach-Oberfrohna (DE)**
• **Morgenstern, Petra
01067 Dresden (DE)**
• **Schreiber, Jürgen, Prof. Dr.
01189 Dresden (DE)**
• **Gerich, Carola
01099 Dresden (DE)**

(74) Vertreter: **Pfenning, Meinig & Partner GbR
Patent- und Rechtsanwälte
An der Frauenkirche 20
01067 Dresden (DE)**

(56) Entgegenhaltungen:
WO-A2-2008/050335     WO-A2-2008/093336
US-A1- 2002 157 613     US-A1- 2008 097 174

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft Verfahren zur Bestimmung des Geschlechts an Vogeleiern. Sie kann für eine Geschlechtsfrüherkennung insbesondere in der Geflügelzucht eingesetzt werden.

Üblicherweise werden Eier nach dem Legen ausgebrütet, was überwiegend in Brutschränken erfolgt, die entsprechend je nach Vogelart über mehrere Tage oder Wochen beheizt werden müssen. Da aber eine Zucht geschlechtsspezifisch durchgeführt wird, ist eine Geschlechtbestimmung nach dem Schlüpfen erforderlich. Diese kann manuell durchgeführt werden, was selbstverständlich einen hohen Aufwand darstellt.

Die geschlüpften Küken mit dem jeweils ungewünschten Geschlecht werden dabei überwiegend getötet, was im Gegensatz zu den Interessen des Tierschutzes steht. Aus diesen Gründen sind verschiedene Möglichkeiten versucht worden, um eine Geschlechtsbestimmung in einem möglichst frühen Stadium mit ausreichender Genauigkeit durchführen zu können.

So wird in DE 10 2007 013 107 A1 vorgeschlagen DNArelevantes Zellmaterial mit Licht zu untersuchen und dabei Molekülschwingungsspektren zu messen, die mit Referenzspektren verglichen werden sollen. Dies soll mittels Raman-, IR- oder Terahertz-Spektrosokopie erfolgen. Die so erhaltenen Ergebnisse weisen, insbesondere bei einer Messung innerhalb eines Eies eine sehr hohe Fehlerquote auf, da alle in einem Ei enthaltenen Inhaltsstoffe einen Einfluss bei diesen Verfahren ausüben. Es sind für die Bestrahlung hohe Intensitäten erforderlich, die zu einer Erwärmung und demzufolge einer Beeinträchtigung der bestrahlten Zellen führen. Die Genauigkeit kann bei diesen Vorgehensweisen nur dadurch erhöht werden, indem bereits über mehrere Tage bebrütete Eier untersucht werden.

Dies trifft ebenfalls auf das in DE 697 11 943 T2 beschriebene Verfahren zu. Dabei soll der in extraembryonaler Flüssigkeit enthaltene Anteil eines geschlechtsspezifischen Hormons bestimmt werden.

Weiterhin sind aus WO 2008/050335 A2 ein Verfahren und eine Vorrichtung bekannt, bei denen eine Anzahl von Molekülen mit einem Lichtstrahl angeregt und deren emittierten Signale detektiert werden. Mit einer entsprechenden Ausführungsform soll die in ovo Geschlechtsbestimmung von Küken durchgeführt werden.

Schließlich ist aus der US 2008/097174 A1 eine Methode bekannt, bei der Hautgewebe mit Anregungslicht bestrahlt wird, so dass die Fluoreszenzlebensdauer sowie die Eigenfluoreszenzspektren gemessen werden können. Anhand der mathematischen Auswertung der Messwerte sollen Modelle konstruiert werden um Beziehungen zwischen Fluoreszenzeigenschaften des untersuchten Gewebes und einem Krankheitsbild (Diabetes) herzustellen.

Es ist daher Aufgabe der Erfindung die Bestimmung des Geschlechts von Vögeln an Eiern mit erhöhter Genauigkeit und geringem Aufwand durchführen zu können.

Erfindungsgemäß wird diese Aufgabe mit einem Verfahren, das die Merkmale des Anspruchs 1 aufweist, gelöst. Vorteilhafte Ausgestaltungen und Weiterbildungen der Erfindung sind mit in untergeordneten Ansprüchen bezeichneten Merkmalen erreichbar.

Bei dem erfindungsgemäßen Verfahren wird mit einer Strahlungsquelle elektromagnetische Strahlung auf die Keimscheibe eines unbebrüteten Eies emittiert. Dabei kann mit der eingesetzten elektromagnetischen Strahlung Eigenfluoreszenz am bestrahlten Bereich der Keimscheibe angeregt werden. Nach einem Abschalten der Strahlungsquelle wird am bestrahlten Bereich der Keimscheibe das Abklingverhalten der durch die elektromagnetische Strahlung angeregten Eigenfluoreszenzintensität zeit- und spektralaufgelöst für mindestens eine Wellenlänge der Eigenfluoreszenz mit einem Detektor erfasst.

Mit den ermittelten Intensitätsmesswerten wird dann die fraktale Dimension $D_F$ berechnet und der Wert der fraktalen Dimension $D_F$ wird dann mit einem art- und geschlechtsspezifischen Grenzwert verglichen. Bei Überschreiten des Grenzwertes wird dann das jeweilige Ei als weiblich und bei Unterschreiten als männlich eingestuft. Die Bestimmungssicherheit des jeweiligen Geschlechts kann dadurch erhöht werden, in dem ein nicht nur die jeweilige Vogelart, sondern auch die jeweilige Rasse berücksichtigender Grenzwert bei der Erfindung berücksichtigt wird.

Nach dem Abschalten der Strahlungsquelle für die elektromagnetische Strahlung zur Anregung der Eigenfluoreszenz nimmt die Intensität der Eigenfluoreszenzstrahlung über die Zeit ab. Es kann von einer Relaxation in den Grundzustand, nach dem Abschalten der Strahlungsquelle ausgegangen werden.

Allgemein kann bei fraktalen Strukturen ein nichtexponentielles Abklingen beobachtet werden, das sich mit folgenden Gleichungen beschreiben lässt:

$$I(t) \approx t^{-\alpha} \qquad\qquad (1)$$

$$D_F = 2 - \frac{\alpha}{2} \qquad\qquad (2)$$

Es können zwei verschiedene Möglichkeiten zur Beschreibung der fraktalen Dimension $D_F$ benutzt werden. Dabei kann die Bestimmung des Maximums der Funktion von Bedeutung sein und berücksichtigt werden. In die Berechnungen können bevorzugt auch noch bestimmte weitere Wellenlängen, die größer oder kleiner als das Maximum sind, einbezogen werden. Diese ausgewählten Wellenlängen können mit konstanter Differenz voneinander abweichen, so dass ein Abstand von 1,5 nm zwischen benachbarten Wellenlängen eingehalten werden kann.

Die detektierten zeitaufgelösten Fluoreszenzspektren werden mit Hilfe der fraktalen Analyse ausgewertet. Durch eine Anregung von Eigenfluoreszenz mit geeigneter Strahlung können unterschiedliche Moleküle gleichzeitig angeregt werden und diese sich gegenseitig durch elektronische, photonische und Schwingungszustände beeinflussen. Zusätzlich können dabei auf Grund von Wechselwirkungen Sättigungszustände auftreten. Dieses komplexe Verhalten kann nicht bzw. nicht ausreichend durch eine Beschreibung mit vielen metastabilen Zuständen und damit auch mehreren Lebensdauern bzw. maximalen Abklingzeiten approximiert werden. Es liegt daher kein einfaches exponentielles Abklingen der Eigenfluoreszenz vor, da die Wechselwirkungen zwischen den Molekülen zu charakteristischen Korrelationen im zeitlichen Abklingen, die auch über die Lebensdauer (Halbwertzeit) hinausführen können. Diese Korrelationen sind charakteristisch für die Gesamtheit der mit der zur Anregung der Eigenfluoreszenz genutzten Strahlung angeregten Moleküle, da sie die Wechselwirkungen zwischen Wirkungen berücksichtigen. Das Verhalten der Gesamtheit kann mit einer lang andauernden Zeit-Zeit-Korrelation modelliert werden, die keine streng exponentielle Form aufweist.

Ein einzelnes Molekül hat eine definierte Elektronenstruktur. Da bei der Gesamtheit der jeweiligen Moleküle auch unterschiedliche Strukturen vorliegen, verliert sich die einfache Übergangsstruktur. Bei zwei Molekülen wird die Addition der Exponentialfunktionen eine neue Exponentialfunktion mit einem Knick ergeben.

Die Abgabe von Energie beim Abfall zu einem niedrigeren S1-Niveau erfolgt dabei nicht als elektromagnetische Strahlung (Licht), sondern die Energie geht zuerst zumindest teilweise auf ein anderes Molekül über und wird dann mit veränderter Energie, da die eigene Energie dieses Moleküls hinzukommt, als elektromagnetische Strahlung (Eigenfluoreszenz) abgegeben. Dieses Verhalten setzt sich von Molekül zu Molekül fort, und je mehr Moleküle vorhanden sind umso komplexer ist dieses Verhalten, so dass im Nachhinein keine Trennung der jeweiligen Eigenfluoreszenz der verschiedenen Moleküle durch Detektion möglich ist.

Dadurch werden aber auch die Wechselwirkungen immer größer und lassen sich nicht mehr exponentiell darstellen. Bei der Analyse des Abklingverhaltens der Eigenfluoreszenz kann man ein exponentielles Verhalten noch feststellen. Mit einem größeren zeitlichen Abstand von einer Anregung konvergiert der Verlauf der Exponentialfunktion nicht mehr. Aber genau dieses nichtexponentielle Abklingverhalten der Eigenfluoreszenz kann mit der Bestimmung der fraktalen Dimension $D_F$ berücksichtigt werden.

Die Bestimmung der fraktalen Dimension $D_F$ kann bei der Erfindung vorzugsweise durch drei Vorgehensweisen durchgeführt werden, auf die nachfolgend näher erklärend eingegangen werden soll.

Die erste Möglichkeit besteht darin, an ein empirisch bestimmtes Modell mit der Gleichung

$$y(t, A, B, C, \alpha, t_0) = A \cdot e^{B(|t-t_0|)^\alpha} + C \qquad (3)$$

eine Anpassung durch Bestimmung der kleinsten Fehlerquadrate durchzuführen. $t_0$ entspricht der Zeit, bei der die maximale Intensität auftritt. Über die Variable $\alpha$ kann die fraktale Dimension $D_F 1$ aus Gleichung (2) berechnet werden, da $y(t, A, B, C, \alpha, t_0)$ analog zu $I(t)$ aus Gleichung (1) verwendet werden kann.

Die Werte der Parameter A, B, C, $\alpha$ und $t_0$ können dabei fiktiv zur Anpassung einer zeitabhängigen Bestimmung von Maxima in iterativer Form mit einer Anpassungsfunktion nach Gleichung (3) bestimmt werden, um eine bestmöglich Anpassung an die experimentell bestimmten Werte zu erreichen.

Bei der zweiten Möglichkeit zur Berechnung der fraktalen Dimension $D_F$ können die kumulativen Eigenschaften ausgenutzt werden. Mit Hilfe der Autokorrelation können Zusammenhänge zwischen den detektierten Intensitäten der Eigenfluoreszenz zu verschiedenen Zeitpunkten beim Abklingen der Eigenfluoreszenzintensität einer Messreihe ermittelt werden. Aus der Funktion y() gemäß (3) kann der jeweils aktuelle Zeitpunkt (t) mit dem Zeitpunkt im Bereich der maximalen Wellenlänge ($t_0$) korreliert werden. Es erfolgt dabei eine Verschiebung des Verlaufs der ermittelten Fluoreszenzintensitäten um eine bestimmte Zeit. Dabei kann dann eine Differenzbildung mit dem so verschobenen Funktionsverlauf und dem nicht bzw. anders zeitlich verschobenen Funktions- bzw. Intensitätsmesswertelaufs durchgeführt werden.

Für die Differenz-Autokorrelationsfunktion C(n) kann das bei kooperativen Fluoreszenz-Vorgängen mögliche Skalengesetz angewendet werden:

$$C(t) \sim t^{2H} \qquad (5)$$

7.582

Der Exponent H oder die Größe $D_F = 2 - H$ (in der Literatur auch als fraktale Dimension der stochastischen Intensitäts-Schwankungen bezeichnet), stellt dabei die charakteristische Größe dar, die mit steigenden Werten eine zunehmende kollektive Wechselwirkung der durch elektromagnetische Strahlung angeregten Elektronen in der Keimscheibe beschreibt. Deshalb wird hier die Kenngröße $D_F$ als Maß zur Unterscheidung der beiden Geschlechter ,männlich' und ,weiblich' verwendet.

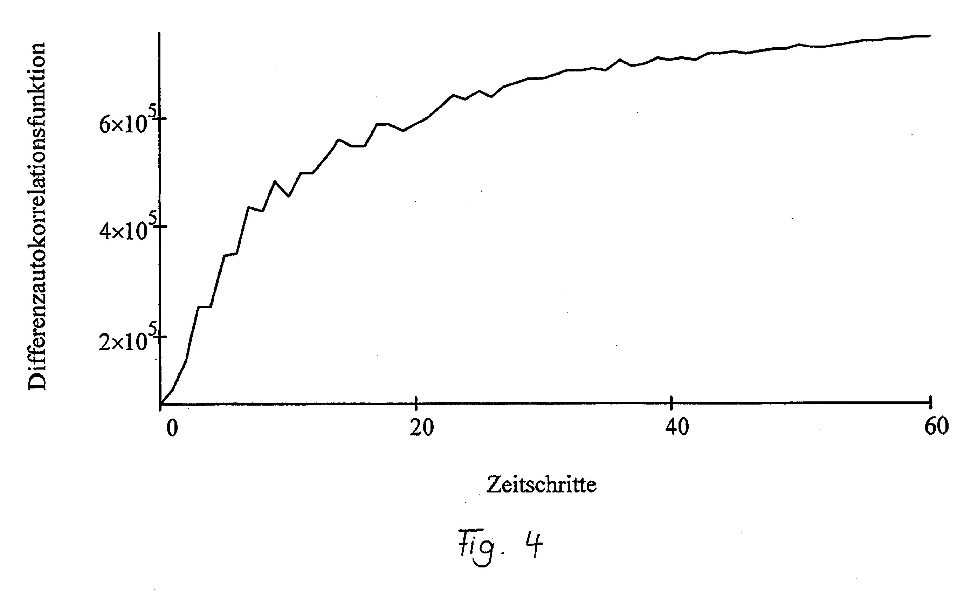

An die neu gewonnene Autokorrelationsfunktion können Geraden angelegt werden, die eine bestimmte Menge an Zeitpunkten einschließen. Man erhält so eine neue Funktion, deren Graph in Figur 4 gezeigt ist.

Mit den berechneten Werten der fraktalen Dimension $D_F1$ und $D_F2$ kann abschließend mit statistischen Methoden (logistische Regression und Diskriminanzanalyse) ein Modell erstellt werden, mit dem eine Klassifikation in die Klassen "männlich" und "weiblich" vorgenommen werden kann. Die Diskriminanzanalyse kann als Kontroll-Verfahren genutzt werden, das die Ergebnisse der logistischen Regression bestätigen kann. Dabei ist $D_F1$ der Wert der fraktalen Dimension, der mit der ersten Möglichkeit unter Nutzung von Gleichung (3) und $D_F2$ der Wert der mit der zweiten Möglichkeit, nämlich der Bestimmung der Differenz- Autokorrelationsfunktion, bestimmt worden ist.

Zusätzlich kann eine Hauptkomponentenanalyse der Eigenfluoreszenzspektren durchgeführt werden. Dieses statistische Verfahren filtert aus der Gesamtinformation eines Spektrums der Eigenfluoreszenz die wesentlichen Informationen heraus. Die gewonnenen Hauptkomponenten können in weiterer Folge mit statistischen Algorithmen, wie z. B. einer Diskriminanzanalyse zur Erstellung eines Klassifikations-Modells verwendet werden.

Bevorzugt sollte die Keimscheibe mit monochromatischer elektromagnetischer Strahlung bestrahlt werden. Für die Bestimmung der Eigenfluoreszenz kann ein Fluoreszenzspektrometer als Detektor eingesetzt werden. Die Bestrahlung mit einem Strahl für die Eigenfluoreszenzanregung sollte so erfolgen, dass der Strahl so auf die Keimscheibe auftrifft, dass keine Strahlung auf derselben Achse zurück reflektiert wird. Die Bestrahlung sollte möglichst in einem Winkel, der mindestens 10 ° von der senkrecht zur bestrahlten Oberfläche der Keimscheibe ausgerichteten Achse abweicht, erfolgen.

Vorteilhaft kann dabei die für die Eigenfluoreszenzanregung eingesetzte elektromagnetische Strahlung mit einer optischen Faser auf die Keimscheibe gerichtet und mit dieser optischen Faser oder einer zweiten optischen Faser die in Folge Eigenfluoreszenz auftretende elektromagnetische Strahlung auf den Detektor gerichtet werden. Zwei optische Fasern können dabei in einem Winkel schräg geneigt zueinander ausgerichtet sein, um eine gegenseitige Beeinflussung der beiden Strahlungen zu vermeiden bzw. zu reduzieren, so dass auf ein optisches Filter vor dem Detektor für die Wellenlänge der Anregungsstrahlung verzichtet werden kann. Bei der Erfassung der Eigenfluoreszenzintensitäten kann jedoch im Strahlengang zwischen dem bestrahlten Bereich der Keimscheibe und dem Detektor ein optisches Filter angeordnet sein, mit dem zumindest die elektromagnetische Strahlung der Wellenlänge, die für die Eigenfluoreszenzanregung eingesetzt wird, gesperrt werden kann, so dass Strahlung mit dieser Wellenlänge nicht bzw. mit einer äußerst kleinen Intensität auf den Detektor auftrifft. Hierfür kann ein Notchfilter eingesetzt werden.

Für die Eigenfluoreszenzanregung sollte elektromagnetische Strahlung aus dem Wellenlängenbereich der UV-Strahlung eingesetzt werden. Diese kann von einer Laserlichtquelle, die bevorzugt gepulst betrieben wird, emittiert werden.

Die Detektion sollte bevorzugt mit bekannter und dabei auch konstanter Abtastrate durchgeführt werden. Dabei sollte die Spektralanalyse jeweils zu gleichen Zeitpunkten durchgeführt werden. Die Abtastrate sollte im Bereich 50 ps bis 1000 ps liegen. Dabei kann nach dem Abschalten der Strahlungsquelle für die Eigenfluoreszenzanregung mit den bekannten Zeitabständen das Abklingen der Eigenfluoreszenzspektren erfasst werden.

Das erfindungsgemäße Verfahren wird in ovo durchgeführt, ohne dass ein Ei zerstört oder etwas aus dem Ei für die Untersuchung entnommen werden muss. Dabei kann eine geeignete Sonde durch die Schale eingeführt und die Bestrahlung der Keimscheibe und auch Detektion durch ein kleines Loch erreicht werden.

Jede Fluoreszenz ist charakteristisch für ein bestimmtes Fluorophor. Auch im Zellmaterial von Keimscheiben sind Stoffe, Substanzen, Moleküle enthalten die als Fluorophor wirken und genutzt werden können. Diese können sich gegenseitig beeinflussen. Das unterschiedliche Fluoreszenzverhalten führt zu einem komplexen nicht-exponentiellen Abklingverhalten. Dieses kann über die fraktale Dimension beschrieben werden.

Mit der Erfindung kann die Geschlechtsbestimmung überraschenderweise an unbebrüteten Eiern mit einer Sicherheit von mehr als 75 % erreicht werden, wodurch der für das Ausbrüten erforderliche Aufwand reduziert und geschlechtsspezifisch vor dem Brüten aussortierte Eier einer anderweitigen sinnvollen Verwertung zugeführt werden können, was natürlich besonders vorteilhaft ist.

Nachfolgend soll die Erfindung beispielhaft näher erläutert werden.

Dabei zeigen:

Figur 1 in schematischer Form die Bestrahlung einer Keimscheibe und die Detektion von Eigenfluoreszenz;

Figur 2 ein Diagramm der Intensitäten detektierter Eigenfluoreszenzspektren zu unterschiedlichen Zeitpunkten;

Figur 3 den zeitlichen Verlauf einer detektierten Eigenfluoreszenzintensität;

Figur 4 den Verlauf einer Differenz-Autokorrelationsfunktion und

Figur 5 den Funktionsverlauf einer aus mehreren Geraden zusammengesetzten Funktionen der Differenz-Autokorrelationsfunktion.

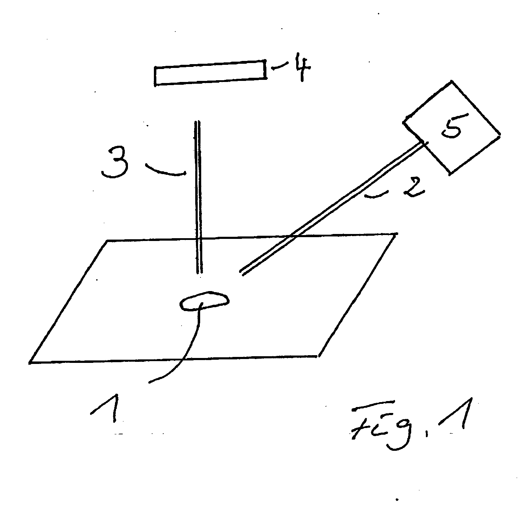

In Figur 1 ist gezeigt, wie Laserstrahlung mit einer Wellenlänge von 337,1 nm, die von einem StickstoffLaser, als Strahlungsquelle 5, zur Anregung von Eigenfluoreszenz an der Keimscheibe 1 emittiert worden ist, mittels der optischen Faser 2 auf eine Keimscheibe 1 gerichtet ist. Die Faser 2 ist in einem Winkel im Bereich 60 ° bis 70° zur Horizontalen bzw. der Ebene der Keimscheibe 1 ausgerichtet.

Die von der Keimscheibe 1 emittierte Eigenfluoreszenzstrahlung kann bei diesem Beispiel mit der zweiten optischen Faser 3 auf einen nicht dargestellten Detektor/Laser-Fluoreszenzspektrometer 4, der/das zu einer Spektralanalyse geeignet ist, gerichtet werden. Die optische Faser 3 ist senkrecht zur Horizontalen bzw. der Ebene der Keimscheibe 1, in der diese angeordnet oder bestrahlt ist, ausgerichtet. Dadurch kann eine Beeinflussung der Detektion durch die Bestrahlung zur Anregung der Fluoreszenz vermieden werden.

Erfolgt die Führung der Strahlung für die Anregung und auch die der Eigenfluoreszenzstrahlung über eine einzige optische Faser oder die Detektion erfolgt unmittelbar mit einem geeigneten Detektor kann ein optisches Filter oder ein entsprechender optischer Strahlteiler vor dem Detektor angeordnet werden, der die Wellenlänge der für die Anregung eingesetzten elektromagnetischen Strahlung sperrt. In diesem Falle ist ein Notchfilter, der für elektromagnetische Strahlung mit der Anregungswellenlänge 337,1 nm nicht transparent ist, geeignet. Seine Sperrwirkung kann dabei in einem Intervall $\pm$ 2 nm um diese Wellenlänge liegen.

Die optischen Fasern 2 und 3 können dabei mit in einem Abstand von 5 mm bis 25 mm zur Oberfläche der Keimscheibe 1 mit ihren in diese Richtung weisenden Stirnenden angeordnet sein.

Wird für die Anregung der Eigenfluoreszenz und die Detektion die jeweilige Strahlung mittels derselben optischen Faser geführt, sollte diese so ausgerichtet sein, wie die in Figur 1 gezeigte optische Faser 2.

Nachfolgend wird auf die Auswertung und die Bestimmung der fraktalen Dimension $D_F$ eingegangen, die an Keimscheiben aus Eiern des normalen Haushuhns (gallus gallus domesticus) durchgeführt worden sind. Dabei wurde für die Anregung ein Stickstofflaser mit einer Pulsenergie 140 $\mu$J und einer Pulsdauer von 0,7 ns eingesetzt. Er konnte bei einer Frequenz im Bereich 2 Hz bis 50 Hz gepulst betrieben werden. Die Detektion erfolgte mit einem Fluoreszenzspektrometer, an dem ein Polychromator und eine ICCD (Intensified Charge-Coupeld Device) vorhanden waren Bei der Detektion wurden die Eigenfluoreszenzintensitäten spektral aufgelöst mit einer konstanten Abtastrate von 250 ps im Wellenlängenbereich zwischen minimal 304,6 nm und maximal 955,3 nm erfasst.

Nach der Bestimmung der fraktalen Dimension $D_F$ an einer Keimscheibe eines Eies erfolgte eine Überprüfung des Bestimmungsergebnisses durch eine Bestimmung unterschiedlicher geschlechtsspezifischer DNA-Anteile, wie dies von T.R. Tiersch in "Idenfication of sex in chickens by flow cytometry"; Word's Poultry Science Journal (2003); Nr. 59; S. 25-32 beschrieben ist.

Es wurden Untersuchungen an 87 Keimscheiben als Proben durchgeführt.

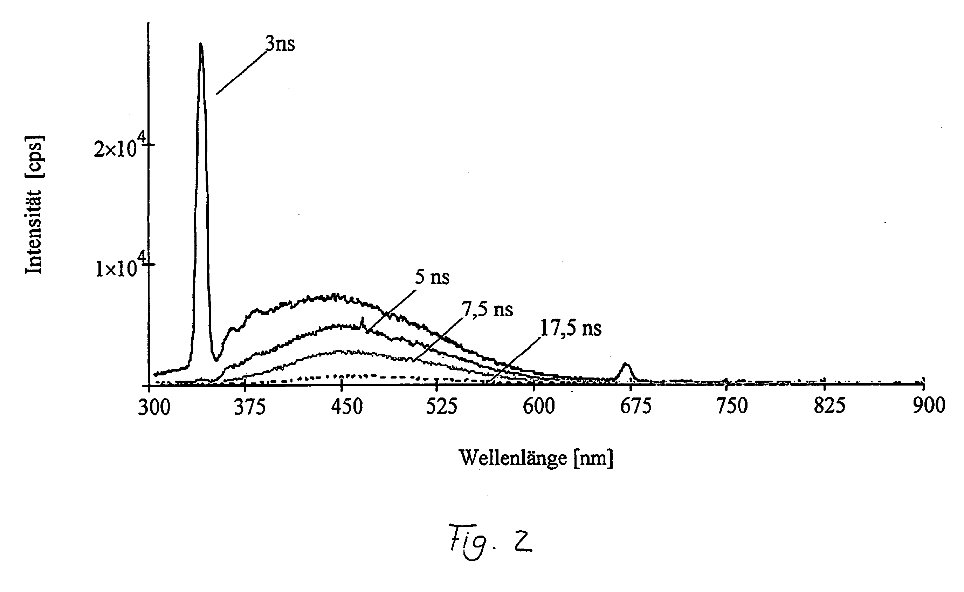

Im in Figur 2 gezeigten Diagramm zeigen die vier Kurvenverläufe detektierter Fluoreszenzintensitäten über einen Wellenlängenbereich von 300 nm bis 900 nm zu verschiedenen Zeitpunkten, die das Abklingverhalten der Eigenfluoreszenzintensitäten spektralaufgelöst wiedergeben. Das Maximum bei ca. 340 nm entspricht der Wellenlänge der für die Eigenfluoreszanzanregung eingesetzten Strahlung.

Es wird deutlich, dass nach 17,5 ns kein signifikanter Nachweis an Eigenfluoreszenz möglich war.

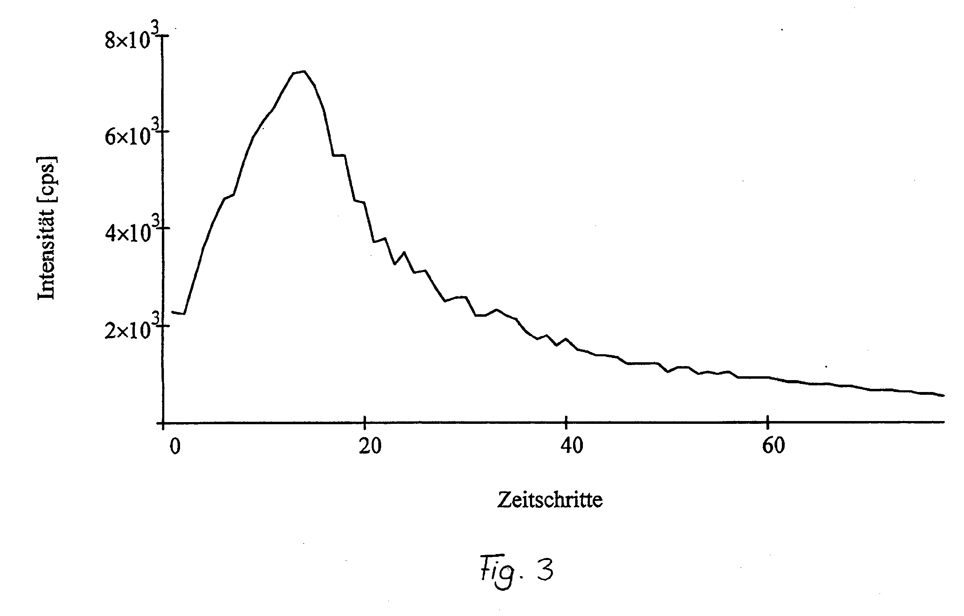

In Figur 3 ist der zeitliche Verlauf, also das Abklingverhalten der Eigenfluoreszenz, von Eigenfluoreszenzintensitäten gezeigt. Dabei wurden dreißig Wellenlängen berücksichtigt, um den Funktionsverlauf einer Glättung zu unterziehen.

Es werden dabei die Eigenfluoreszenzintensitäten eines ausgewählten Wellenlängenbereichs, in dem die maximale Eigenfluoreszenzintensität enthalten ist, berücksichtigt.

Die Angabe der Zeitschritte berücksichtigt die Abtastrate von 250 ps, so dass die 250 ps einen Zeitschritt darstellen.

Mit dem in Figur 4 gezeigten Diagramm ist die bestimmte Differenzautokorrelationsfunktion kenntlich gemacht. Dabei wurde eine zeitliche Verschiebung durchgeführt, um den Bereich auszublenden, in dem noch zur Eigenfluoreszenzanregung bestrahlt worden ist.

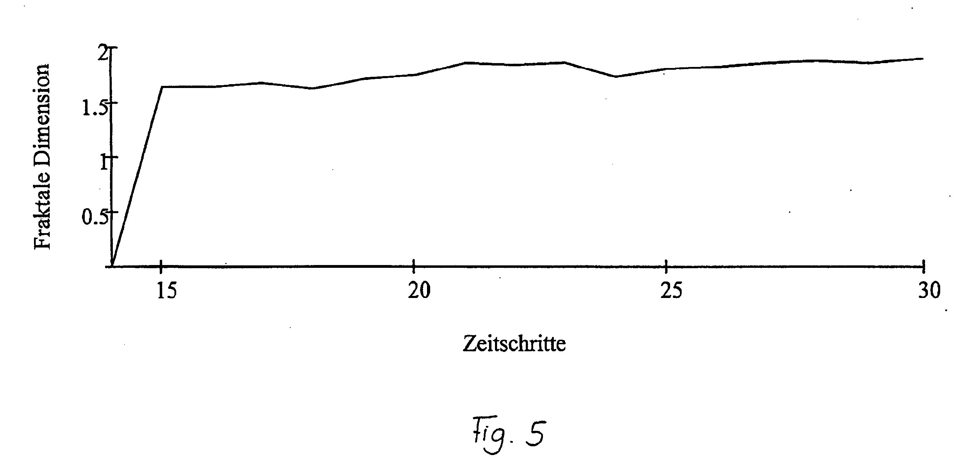

Bei dem in Figur 5 gezeigten Diagramm wurde eine Glättung der Differenzautokorrelation durchgeführt, indem eine Mittelwertbildung in Zeitintervallen erfolgte, die zu zeitlich erweiterten Bereichen mit gleichen Funktionsanstiegen führte.

Als Grenzwert für die fraktale Dimension $D_F 1$ konnte bei den durchgeführten mit der ersten Möglichkeit über das empirische Modell unter Nutzung der Gleichung (3) der Wert 1,54 und mittels der zweiten Möglichkeit (Differenz-

autokorrelationsanalyse) der Wert $D_F2$ zu 1,506 ermittelt werden. Demzufolge waren Eier mit Keimscheiben, an denen die bestimmte fraktale Dimension $D_F$, jeweils größer als der entsprechende Grenzwert war, als weiblich zu klassifizieren.

Wie in der allgemeinen Beschreibung bereits zum Ausdruck gebracht, kann eine Geschlechtsbestimmung mit einer der beiden erläuterten Möglichkeiten für die Bestimmung der fraktalen Dimension des zeitlichen Abklingverhaltens der Eigenfluoreszenzintensitäten, also mit $D_F1$ oder $D_F2$ durchgeführt werden. Die Bestimmungssicherheit lässt sich jedoch durch Berücksichtigung der mit beiden Möglichkeiten bestimmten Werte von $D_F$ erhöhen. Treffen also die Grenzwertüberschreitung sowohl für $D_F1$ und $D_F2$ zu, ist die Wahrscheinlichkeit, der Richtigkeit der durchgeführten Geschlechtsbestimmung eines weiblichen Eies höher.

## Patentansprüche

1. Verfahren zur in ovo Bestimmung des Geschlechts an Vogeleiern, **dadurch gekennzeichnet, dass** mit einer Strahlungsquelle elektromagnetische Strahlung auf die Keimscheibe (1) eines unbebrüteten Eies emittiert und nach einem Abschalten der Strahlungsquelle (5) am bestrahlten Bereich der Keimscheibe das Abklingverhalten der durch die elektromagnetische Strahlung angeregten Eigenfluoreszenzintensität zeit- und spektralaufgelöst für mindestens eine Wellenlänge der Eigenfluoreszenz mit einem Detektor (4) erfasst und mit den ermittelten Intensitätsmesswerten die fraktale Dimension $D_F$ berechnet wird und der Wert der fraktalen Dimension $D_F$ mit einem art- und geschlechtsspezifischen Grenzwert verglichen wird; wobei bei Überschreiten des Grenzwertes das jeweilige Ei als weiblich und bei Unterschreiten als männlich eingestuft wird.

2. Verfahren nach Anspruch 1, **dadurch gekennzeichnet, dass** die fraktale Dimension $D_F1$ durch Ermittlung der kleinsten Fehlerquadrate bestimmt wird.

3. Verfahren nach Anspruch 1, **dadurch gekennzeichnet, dass** die fraktale Dimension $D_F2$ durch Ermittlung der Differenz-Autokorrelations-Funktion C(t) des Eigenfluoreszenzintensitätsabklingverhaltens bestimmt wird.

4. Verfahren nach einem der vorhergehenden Ansprüche, **dadurch gekennzeichnet, dass** die Keimscheibe (1) mit monochromatischer elektromagnetischer Strahlung bestrahlt und für die Bestimmung der Eigenfluoreszenz ein Fluoreszenzspektrometer (4) eingesetzt wird, wobei die Bestrahlung so erfolgt, dass der Strahl für die Eigenfluoreszenzanregung so auf die Keimscheibe auftrifft, dass keine Strahlung auf derselben Achse reflektiert wird.

5. Verfahren nach einem der vorhergehenden Ansprüche, **dadurch gekennzeichnet, dass** die Detektion mit bekannter konstanter Abtastrate durchgeführt wird.

6. Verfahren nach Anspruch 5, **dadurch gekennzeichnet, dass** die Detektion mit konstanter Abtastrate im Bereich 50 ps bis 1000 ps durchgeführt wird.

7. Verfahren nach einem der vorhergehenden Ansprüche, **dadurch gekennzeichnet, dass** für die Fluoreszenzanregung elektromagnetische Strahlung aus dem Wellenlängenbereich der UV-Strahlung eingesetzt wird.

8. Verfahren nach einem der vorhergehenden Ansprüche, **dadurch gekennzeichnet, dass** die für die Fluoreszenzanregung eingesetzte elektromagnetische Strahlung mittels einer ersten optischen Faser (2) auf die Keimscheibe (1) und die in Folge Eigenfluoreszenz auftretende elektromagnetische Strahlung über eine zweite optische Faser (3) auf den Detektor (4) gerichtet wird und dabei die beiden optischen Fasern (2 und 3) in einem schräg geneigten Winkel zueinander ausgerichtet sind.

## Claims

1. A method for the *in ovo* determination of the sex of birds' eggs, **characterised in that**, using a radiation source, electromagnetic radiation is emitted onto the blastodisc (1) of an unincubated egg, and once the radiation source (5) has been switched off the decay behaviour of the intrinsic fluorescence intensity excited by the electromagnetic radiation is detected in time-resolved and spectrally-resolved manner on the irradiated region of the blastodisc for at least one wavelength of the intrinsic fluorescence with a detector (4), and the fractal dimension $D_F$ is calculated with the established measured intensity values and the value of the fractal dimension $D_F$ is compared with a species-specific and sex-specific limit value; with, if the value exceeds the limit value, the respective egg being classified as female, and if it does not reach said value, being classified as male.

2. A method according to Claim 1, **characterised in that** the fractal dimension $D_F1$ is determined by establishing the least error squares.

3. A method according to Claim 1, **characterised in that** the fractal dimension $D_F2$ is determined by establishing the difference autocorrelation function C(t) of the intrinsic fluorescence intensity decay behaviour.

4. A method according to one of the preceding claims, **characterised in that** the blastodisc (1) is irradiated with monochromatic electromagnetic radiation and a fluorescence spectrometer (4) is used for determining the intrinsic fluorescence, the irradiation taking place such that the beam for exciting the intrinsic fluorescence strikes the blastodisc such that no radiation is reflected on the same axis.

5. A method according to one of the preceding claims, **characterised in that** the detection is carried out with a known constant sampling rate.

6. A method according to Claim 5, **characterised in that** the detection is carried out with a constant sampling rate in the range 50 ps to 1000 ps.

7. A method according to one of the preceding claims, **characterised in that** electromagnetic radiation from the wavelength range of UV radiation is used for exciting the fluorescence.

8. A method according to one of the preceding claims, **characterised in that** the electromagnetic radiation used for exciting the fluorescence is directed onto the blastodisc (1) by means of a first optical fibre (2) and the electromagnetic radiation occurring as a result of intrinsic fluorescence is directed onto the detector (4) via a second optical fibre (3), and in so doing the two optical fibres (2 and 3) are oriented at an obliquely inclined angle to one another.

**Revendications**

1. Procédé permettant la détermination in ovo du sexe des oeufs d'oiseaux,
   **caractérisé en ce que**
   avec une source de rayonnement, un rayonnement électromagnétique est émis sur le disque germinatif (1) d'un oeuf non incubé et, après la déconnexion de la source de rayonnement (5) et **en ce qu'**au niveau de la zone du disque germinatif le comportement en décroissance de l'intensité de fluorescence propre induite par le rayonnement électromagnétique est détectée avec un détecteur (4) selon une résolution temporelle et une résolution spectrale pour au moins une longueur d'onde de la fluorescence propre, et
   la dimension fractale $D_F$ est calculée avec les valeurs de mesure d'intensité détectées, et la valeur de la dimension fractale $D_F$ est comparée à un valeur limite spécifique au type et au sexe ; si ladite valeur est supérieure à la valeur limite, l'oeuf concerné est classé comme étant de sexe féminin et si ladite valeur est inférieure à la valeur limite, il est classé comme étant de sexe masculin.

2. Procédé selon la revendication 1, **caractérisé en ce que** la dimension fractale $D_F1$ est déterminée par le calcul des moindres carrés.

3. Procédé selon la revendication 1, **caractérisé en ce que** la dimension fractale $D_F2$ est déterminée par le calcul de la fonction d'autocorrélation de différence C(t) du comportement en décroissance de l'intensité de fluorescence propre.

4. Procédé selon l'une quelconque des revendications précédentes, **caractérisé en ce que** le disque germinatif (1) est irradié par un rayonnement électromagnétique monochrome, et **en ce qu'**un spectromètre de fluorescence (4) est utilisé pour déterminer la fluorescence propre, l'irradiation étant effectuée de telle sorte que le rayon qui induit la fluorescence propre rencontre le disque germinatif de telle sorte qu'aucun rayonnement n'est réfléchi sur le même axe.

5. Procédé selon l'une- quelconque des revendications précédentes, **caractérisé en ce que** la détection est effectuée à une vitesse de balayage constante et connue.

**6.** Procédé selon la revendication 5, **caractérisé en ce que** la détection est effectuée à une vitesse de balayage constante comprise dans la plage de 50 ps à 1000 ps.

**7.** Procédé selon l'une quelconque des revendications précédentes, **caractérisé en ce qu'**un rayonnement électromagnétique compris dans la plage des longueurs d'ondes du rayonnement UV est utilisé pour l'induction de la fluorescence.

**8.** Procédé selon l'une quelconque des revendications précédentes, **caractérisé en ce que** le rayonnement électromagnétique utilisé pour l'incitation à la fluorescence est dirigé au moyen d'une première fibre optique (2) sur le disque germinatif (1) et le rayonnement électromagnétique produit à la suite de la fluorescence propre est dirigé au moyen d'une deuxième fibre optique (3) sur le détecteur (4) et, en l'occurrence, les deux fibres optiques (2 e 3) étant orientées en formant l'une avec l'autre un angle incliné.

Fig. 1

Fig. 2

*Fig. 3*

Fig. 4

*Fig. 5*

EP 2 336 751 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102007013107 A1
- DE 69711943 T2
- WO 2008050335 A2
- US 2008097174 A1

**In der Beschreibung aufgeführte Nicht-Patentliteratur**

- **T.R. TIERSCH.** Idenfication of sex in chickens by flow cytometry. *Word's Poultry Science Journal,* 2003, vol. 59, 25-32